(19) Europäisches Patentamt
European Patent Office
Office européen des brevets

(11) **EP 3 118 611 B1**

(12) **FASCICULE DE BREVET EUROPEEN**

(45) Date de publication et mention
de la délivrance du brevet:
**05.12.2018 Bulletin 2018/49**

(51) Int Cl.:
***G01N 25/18*** *(2006.01)*     ***G01N 27/18*** *(2006.01)*
***G01N 33/00*** *(2006.01)*

(21) Numéro de dépôt: **16179042.3**

(22) Date de dépôt: **12.07.2016**

(54) **DISPOSITIF D'ANALYSE PERMETTANT L'ANALYSE D'UN MELANGE GAZEUX**

ANALYSEVORRICHTUNG ZUR ANALYSE EINER GASMISCHUNG

ANALYSIS DEVICE FOR ANALYSING A MIXTURE OF GASES

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **16.07.2015 FR 1556724**

(43) Date de publication de la demande:
**18.01.2017 Bulletin 2017/03**

(73) Titulaire: **Commissariat à l'énergie atomique
et aux énergies alternatives
75015 Paris (FR)**

(72) Inventeurs:
• **BOURLON, Bertrand
38950 SAINT MARTIN LE VINOUX (FR)**
• **PHAM HO, Bao-An
38000 GRENOBLE (FR)**

(74) Mandataire: **Brevalex
95, rue d'Amsterdam
75378 Paris Cedex 8 (FR)**

(56) Documents cités:
**DE-B3-102013 103 388**    **FR-A1- 2 995 691
US-A- 4 164 862**    **US-A1- 2012 024 043**

• **UDINA S ET AL: "A micromachined
thermoelectric sensor for natural gas analysis:
Multivariate calibration results", SENSORS AND
ACTUATORS B: CHEMICAL: INTERNATIONAL
JOURNAL DEVOTED TO RESEARCH AND
DEVELOPMENT OF PHYSICAL AND CHEMICAL
TRANSDUCERS, ELSEVIER S.A, CH, vol. 166, 29
novembre 2011 (2011-11-29), pages 338-348,
XP028486933, ISSN: 0925-4005, DOI:
10.1016/J.SNB.2011.11.086 [extrait le 2012-03-01]**

**Description**

**DOMAINE TECHNIQUE ET ÉTAT DE LA TECHNIQUE ANTÉRIEURE**

La présente invention se rapporte à un capteur de gaz permettant l'analyse d'un mélange d'au moins deux gaz, en particulier la mesure de la concentration des composants gazeux dans un mélange d'au moins deux gaz.

La conductivité thermique d'un mélange de gaz dépend de sa composition. Ainsi suivant la concentration en chacun des gaz, la conductivité thermique du mélange varie. Cette propriété est utilisée pour réaliser des capteurs de gaz. La mesure de la conductivité thermique permet de remonter directement à la composition d'un mélange de gaz binaire de gaz de nature connue.

En effet, en considérant un mélange de gaz A1 et A2 et en notant :

$x1$, $x2$ les fractions molaires respective en gaz A1, A2, et $\lambda(x_1, x_2, T_0, P_0)$ la conductivité thermique du mélange de gaz avec les fractions molaires $x_1$, $x_2$, à la température $T_0$ et à la pression $P_0$.

Nous avons :

$$\begin{cases} x_1 + x_2 = 1 \\ \lambda(x_1, x_2, T_0, P_0) = m1 \end{cases}$$

En effet la somme de toutes les fractions de gaz est égale à 1. Pour une température et pression de gaz fixés, la conductivité thermique du mélange ne dépend alors que d'un paramètre $x_1$.

En faisant l'hypothèse que la conductivité thermique en fonction de la composition du mélange est connue et monotone, la mesure de celle-ci permet alors de remonter à la fraction molaire x1 et donc la composition du mélange.

En revanche, cette méthode n'est pas applicable lorsqu'il y a plus de deux gaz. Si on note x1,..., xn les fractions molaires respectives en gaz A1,...,An, une seule mesure ne permet pas de déterminer x1, ..., xn et donc la composition du mélange. Il est en effet possible que deux mélanges ou plus ayant des valeurs x1, ..., xn différentes aient la même conductivité thermique. Il n'est alors pas possible de choisir entre les mélanges

Or la conductivité thermique dépend de la température. Il est donc possible en faisant des mesures à plusieurs températures du mélange gazeux d'obtenir n équations, et donc n-1 mesures pour déterminer x1, ..., xn.

Le document "A micromachined thermoelectric sensor for natural gas analysis: Thermal model and experimental results" Udina, S., Carmona, M., Caries, G., Santander, J., Fonseca, L., Marco, S. (2008) Sensors and Actuators, B: Chemical, 134 (2), pp. 551-558 décrit un capteur permettant de mesurer la concentration de plusieurs gaz. Ce capteur met en oeuvre une membrane suspendue à un substrat en silicium et un moyen de chauffage en polysilicium disposé sur la membrane. La membrane est disposée dans une chambre contenant un mélange de gaz. Une tension électrique est appliquée aux bornes du moyen de chauffage afin qu'il chauffe la membrane. Des échanges thermiques ont lieu avec le mélange de gaz. Une thermopile mesure la température de la membrane, celle-ci variant en fonction de la composition du mélange gazeux. Différentes tensions électriques sont appliquées au moyen de chauffage pour effectuer plusieurs mesures à des températures différentes.

La détermination de la composition du mélange gazeux est relativement longue car elle demande de faire successivement autant de mesures à des températures différentes que de gaz contenus dans le mélange, d'autant plus qu'il est indiqué qu'environ 15 minutes sont requises avant chaque mesure pour s'assurer d'avoir une concentration stationnaire dans la chambre.

Le document US 4 164 862 décrit également un dispositif pour mesurer la composition d'un mélange de N gaz. Celui-ci comporte N chambres de mesure alimentées en parallèle par le mélange de gaz, chaque chambre étant à une température différente. Pour mesurer la conductivité thermique, chaque chambre comporte une résistance électrique faisant partie d'un pont, qui est soumise au flux de gaz. La variation de la valeur de la résistance varie en fonction de la température qui varie en fonction de la composition en gaz. La résistance est alimentée en courant qui, par effet Joule, libère de la chaleur. Du fait de l'écoulement du gaz, la température de la résistance diminue, cette diminution étant fonction de la conductivité thermique du gaz et donc de sa composition. Une boucle de rétroaction est prévue pour modifier l'intensité du courant circulant dans la résistance et ceci en fonction de la température de la résistance. La commande de rétroaction est donc proportionnelle à la conductivité thermique du mélange de gaz.

Ce dispositif est relativement encombrant puisqu'il faut autant de chambres que de composés dans le mélange gazeux.

**EXPOSÉ DE L'INVENTION**

C'est par conséquent un but de la présente invention d'offrir un dispositif d'analyse pouvant analyser un mélange gazeux comportant au moins deux gaz offrant une analyse rapide et un encombrement réduit.

Le but précédemment énoncé est atteint par dispositif d'analyse d'un mélange de n gaz, comportant un canal microfluidique dans lequel est destiné à s'écouler le mélange gazeux à analyser, au moins n-1 éléments suspendus dans le canal de sorte que les n-1 éléments suspendus soient à des positions différentes dans le canal. Le dispositif comporte également des moyens de chauffage de sorte à chauffer chaque volume de mélange gazeux qui entoure chaque élément suspendu à une température donnée différente de celles des autres volumes de mélange gazeux, et des moyens de mesure de la température de chaque élément suspendu ou d'un paramètre dépendant de la conductivité thermique du mélange gazeux.

Les inventeurs ont constaté qu'il était possible, en utilisant des éléments suspendus micrométriques dans un canal microfluidique, d'une part de thermaliser individuellement à une température donnée rapidement un volume du mélange de gaz entourant chaque élément suspendu et, ensuite de mesurer les échanges thermiques entre chaque élément suspendu et le volume de gaz thermalisé à la température donnée. Ainsi les inventeurs obtiennent simultanément des mesures à différents températures. L'analyse est alors rapide et le dispositif présente un encombrement réduit. En outre les volumes de gaz à thermaliser étant très faibles, le dispositif nécessite une faible puissance pour son fonctionnement.

Du fait de la petite taille du canal, le remplissage du canal et l'homogénéisation du mélange gazeux dans le canal sont rapides.

En d'autres termes, on crée à l'échelle d'un élément suspendu un environnement gazeux à une température donnée et on mesure les échanges thermiques entre l'élément suspendu et l'environnement gazeux à cette température donnée, ce qui permet de déterminer, dans un mode de réalisation, la conductivité thermique du mélange gazeux à cette température. En utilisant plusieurs éléments suspendus dans un environnement gazeux à différentes températures, on détermine les conductivités thermiques du même mélange mais à des températures différentes, ce qui permet de remonter aux concentrations de chaque constituant du mélange gazeux, ces constituants étant connus. La mesure de la conductivité thermique à une température donnée peut être faite avec des moyens connus, par exemple chaque élément suspendu peut faire partie d'un détecteur de conductivité thermique ou TCD ("Thermal Conductivity Detector" en terminologie anglo-saxonne).

Dans un mode de réalisation, chaque volume de gaz comporte un élément suspendu. La taille des éléments suspendus et leur disposition les uns par rapport aux autres sont telles qu'elles assurent qu'ils n'aient pas ou peu d'influence thermique les unes sur les autres. Dans un exemple de réalisation, les éléments sont formés par des membranes suspendues de dimensions micrométriques, et au moins un conducteur électrique sur une face de la membrane forme à la fois les moyens de chauffage du mélange gazeux et les moyens de mesure de la température de la membrane, par exemple en mesurant la variation de résistance électrique du conducteur électrique qui dépend de la température. Dans un autre exemple de réalisation, les éléments sont formés par des fils de dimensions micrométriques tendus transversalement dans le canal et formant directement les moyens de chauffage par effet Joule et de mesure de la température. En variante, les moyens de chauffage peuvent être un fil suspendu à distance de l'élément suspendu.

Dans un autre mode de réalisation, on utilise, des moyens de chauffage communs aux différents éléments suspendus qui forment les moyens de mesure.

La mesure de la variation des résistances électriques du conducteur électrique ou du fil suspendu permet de remonter à la composition du mélange gazeux.

En variante ou de manière additionnelle, on peut mesurer la conductance thermique et/ou la capacité thermique et/ou tout autre paramètre physique dépendant de la conductance thermique et/ou de la capacité thermique, par exemple une température, une résistance, une tension, un courant.

La présente invention a alors pour objet un dispositif d'analyse selon la revendication 1.

Dans un premier mode de réalisation, chaque volume de mélange gazeux comporte ses moyens de chauffage individuels.

Par exemple, le conduit a une section transversale rectangulaire présentant une hauteur et une largeur, la distance entre deux bords en regard de deux éléments suspendus successifs est de l'ordre de la hauteur du canal. La distance entre deux bords en regard de deux éléments suspendus successifs peut être comprise entre $200 \mu m$ et $2000 \mu m$.

Dans un exemple de réalisation, chaque élément suspendu peut comporter une membrane suspendue par des bras de suspension dans le canal et les moyens de chauffage peuvent comporter un fil conducteur formé sur une face de la membrane. Par exemple, les membranes ont des surfaces comprises entre 200 $\mu m^2$ et 200000 $\mu m^2$.

De manière avantageuse, les bras de suspension ont une longueur de plusieurs dizaines de $\mu m$ et une largeur de quelques $\mu m$ à quelques dizaines de $\mu m$.

Dans un autre exemple de réalisation, chaque élément suspendu comporte au moins un fil suspendu. Le fil suspendu peut former les moyens de chauffage et les moyens de mesure de température ou de la variation de température.

Dans un exemple de réalisation, le dispositif d'analyse peut comporter n-1 puces comportant un sous-canal fluidique disposées dans le canal fluidique du dispositif, le volume intérieur des sous-canaux fluidiques étant destinés à contenir une zone de mélange gazeux, chaque élément suspendu étant suspendu dans un sous-canal fluidique, les moyens de chauffage étant situés sur une face extérieure de chaque puce.

Dans un deuxième mode de réalisation, les moyens de chauffage sont communs à tous les volumes de mélange gazeux. Les moyens de chauffage peuvent comporter au moins un fil conducteur destiné à chauffer tous les volumes de mélange gazeux. Par exemple, les moyens de chauffage sont disposés de part et d'autres des éléments suspendus de sorte à imposer un gradient thermique au mélange gazeux situés entre les moyens de chauffage.

De préférence, le conduit a une section transversale rectangulaire présentant une hauteur et une largeur et dans lequel la distance entre les moyens de chauffage et les moyens de mesure est inférieure ou à la hauteur du canal.

Selon une caractéristique additionnelle du deuxième mode de réalisation, les moyens de chauffage et les moyens de mesure comportent des fils conducteurs électriques suspendus dans le canal. Le fil suspendu a par exemple une section transversale comprise entre 1 $\mu$m et 20 $\mu$m et une longueur comprise entre 100 $\mu$m et 1000 $\mu$m.

De manière avantageuse, la section du canal fluidique est comprise entre 100 $\mu$m$^2$ et 1 mm$^2$.

Chaque élément suspendu peut être monté en pont de Wheatstone avec au moins un autre élément suspendu disposé à l'extérieur du canal microfluidique.

Selon une caractéristique additionnelle, le système de contrôle et de mesure de la température peut comporter des moyens d'alimentation électrique aptes à appliquer un signal continu permettant le chauffage du mélange gazeux et un signal alternatif permettant la mesure de la température ou variation de température.

La présente invention a également pour objet un procédé d'analyse d'un mélange gazeux de n gaz selon la revendication 14.

Selon un exemple, l'étape b) a lieu en chauffant l'élément suspendu à une température proche de celle à laquelle est chauffé le volume de gaz entourant ledit élément suspendu.

Selon un autre exemple, l'étape a) a lieu en appliquant une tension continu à un conducteur électrique et dans lequel l'étape b) a lieu en appliquant une tension alternative au conducteur électrique ou à un autre conducteur électrique.

**BRÈVE DESCRIPTION DES DESSINS**

La présente invention sera mieux comprise sur la base de la description qui va suivre et des dessins en annexe sur lesquels:

- la figure 1 est une vue en coupe longitudinale représentée schématiquement d'un exemple de dispositif d'analyse d'un mélange gazeux selon un premier mode de réalisation,
- la figure 2 est une vue de dessus représentée schématiquement d'un exemple d'un élément suspendu dans le canal microfluidique,
- la figure 3 est une représentation graphique de la variation de la conductivité thermique en mW/mK d'un mélange gazeux en fonction de la température,
- la figure 4 est une représentation d'un élément suspendu et du volume de gaz échauffé qui l'entoure,
- les figures 5A à 5K sont des représentations schématiques d'étapes d'un exemple procédé de réalisation d'un dispositif d'analyse selon l'invention,
- la figure 6 est une représentation schématique d'un exemple d'un dispositif d'analyse selon un deuxième mode de réalisation,
- la figure 7 est une représentation des données expérimentales de calibration d'un dispositif selon l'invention avec mélange de $CO_2$, He, $N_2$,
- la figure 8 est une représentation graphique d'une interpolation d'ordre 2 des données de la figure 8,
- la figure 9 est une mesure expérimentale d'une mesure expérimentale faite avec un dispositif selon l'invention sur un mélange de 5% de $CO_2$, 15% d'Hélium, et 81.5% d'azote.

**EXPOSÉ DÉTAILLÉ DE MODES DE RÉALISATION PARTICULIERS**

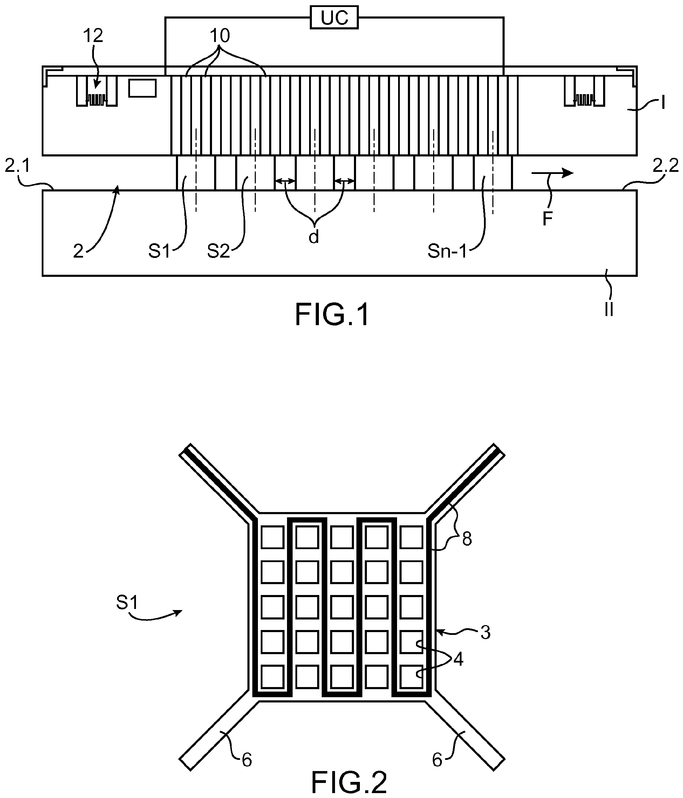

Sur la figure 1, on peut voir une représentation schématique d'un premier mode de réalisation d'un dispositif d'analyse d'un mélange gazeux selon l'invention. Le mélange gazeux comporte n gaz, n étant un entier positif > 1. Le plan de coupe est horizontal.

Le dispositif comporte un canal microfluidique 2 comportant une extrémité d'alimentation 2.1 destinée à être connectée à une alimentation en mélange gazeux à analyser et une extrémité d'évacuation 2.2. Le mélange gazeux à analyser est donc destiné à s'écouler dans le canal dans la direction symbolisée par la flèche F, au moins lors du remplissage. Le canal est par exemple réalisé en deux parties, par exemple par gravure de deux substrats en matériau semi-conducteur, par exemple en silicium ou d'un substrat en semi-conducteur et un substrat en verre. Le canal a dans cet exemple une section transversale rectangulaire.

Comme nous le verrons par la suite, l'analyse peut se faire alors que le mélange gazeux est immobile, dans ce cas on peut prévoir que le canal ne comporte qu'une extrémité ouverte par laquelle a lieu l'alimentation et l'évacuation en mélange gazeux.

En variante encore, le canal peut comporter plus de deux ouvertures. Par exemple, le dispositif peut comporter un capot poreux ou un capot muni de plusieurs trous, le capot permettant l'échange sans pompe avec le gaz extérieur à la cavité, par exemple dans des applications de type téléphonie mobile dans lesquelles la mise en oeuvre d'une pompe n'est pas envisageable.

Dans l'exemple représenté, le canal 2 est rectiligne et s'étend le long d'un axe longitudinal X. Il sera compris que le canal peut présenter tout profil, par exemple courbé ou en spiral.

Le dispositif comporte également au moins n-1 éléments suspendus S1, S2...Sn-1 dans le canal 2, le nombre d'élément suspendus étant au moins égal à 2. Le dispositif est capable d'analyser le mélange gazeux comportant n gaz différents.

Les éléments sont disposés à des emplacements différents le long de la direction d'écoulement F, i.e. les éléments S1, S2...Sn-1 sont disposés les uns à la suite des autres à distance les uns des autres de sorte à occuper chacun un tronçon du canal 2. Dans l'exemple représenté, les éléments suspendus sont séparés d'une distance d constante. En variante, cette distance pourrait variée entre deux éléments suspendus et les suivants.

Dans le cas d'un canal rectiligne, les éléments suspendus ont chacun un emplacement donné le long de l'axe longitudinal.

Sur la figure 2, on peut voir un exemple d'un élément pouvant S1 être suspendu dans le canal 2. Il s'agit d'une membrane 3 de forme carrée ou rectangulaire et avantageusement percée de trous 4. La membrane 3 est suspendue au canal au niveau de ses quatre sommets par des bras 6. Les trous 4 ont pour avantage d'une part d'aider à la libération de la membrane lors des différents procédés de fabrication et d'autre part de réduire la capacité thermique de la membrane. Avantageusement, les bras ont tous la même longueur. De préférence, les bras présentent une section faible et/ou une longueur importante pour limiter les fuites thermiques de la membrane vers le substrat. Une membrane pleine et/ou de forme différente et/ou suspendue par deux ou trois bras ou plus de quatre bras ne sort pas du cadre de la présente invention.

De préférence, la membrane est disposée parallèlement au fond plan du canal.

Le dispositif comporte des moyens de chauffage du volume du mélange gazeux entourant chaque élément suspendu S1, S2...Sn-1 à des températures T1, T2,...Tn-1 différentes les unes des autres.

Le dispositif comporte également des moyens M pour mesurer les échanges thermiques entre l'élément suspendu Si et le volume de gaz à la température Ti, $1 \leq i \leq n-1$. La mesure de ces échanges thermiques permet de remonter à la conductivité thermique du mélange gazeux à la température Ti ou à tout paramètre dépendant de la conductivité thermique.

Sur la figure 2, les moyens de chauffage sont formés par un conducteur électrique thermorésistif 8 formé sur l'une des faces de la membrane. Il s'étend entre deux bras de suspension 6 et chemine sur la membrane de sorte à en occuper la surface de l'une des faces de manière sensiblement uniforme. Dans l'exemple représenté, le conducteur 8 est en forme de créneaux.

Les moyens de mesure de la température ou de la variation de température utilisent le conducteur électrique permettant de mesurer les échanges thermiques entre l'élément suspendu et le mélange gazeux, qui sont représentatifs de la conductivité thermique du mélange gazeux à la température donnée.

Le conducteur électrique est connecté à des moyens d'alimentation électrique en tension ou en courant et à des moyens de mesure en courant ou en tension respectivement.

Les moyens de mesure mesurent par exemple une tension aux bornes du conducteur électrique ou le courant circulant dans le conducteur électrique.

Dans un exemple de fonctionnement, les moyens de chauffage appliquent une tension continue et les moyens de mesure mesurent un courant qui est représentatif de la température de la membrane et donc des échanges thermiques entre la membrane et l'environnement gazeux.

Dans un autre exemple de fonctionnement, les moyens de chauffage appliquent au fil conducteur une tension continue pour chauffer le volume de gaz Vi à Ti et les moyens de mesure appliquent un signal alternatif au fil conducteur permettant de mesurer la conductivité thermique en appliquant des excursions de température à la membrane et en mesurant les échanges thermiques entre la membrane et le volume de gaz.

Chaque élément conducteur est connecté individuellement aux moyens d'alimentation électrique et aux moyens de mesure.

L'élément conducteur 8 est directement en contact avec la membrane, sa résistance va directement dépendre de la température de la membrane. En mesurant la variation de tension due à la variation de la résistance électrique du conducteur électrique à Ti, on peut remonter à la variation de la température de la membrane qui dépend de la conductivité thermique du mélange gazeux à Ti et donc de sa composition.

En variante, le dispositif pourrait comporter une pluralité de puces comprenant chacune un canal et une membrane suspendue dans le canal microfluidique 2. En outre chaque puce comporterait son moyen de chauffage, par exemple une résistance située en face arrière de la puce. Les moyens de chauffage chaufferaient la puce et le gaz contenu dans le canal. Chaque membrane comporterait par exemple un conducteur auquel on imposerait une tension continue ou alternative ou un courant continu ou alternatif faible, pour mesurer les échanges thermiques entre les membranes et le gaz. Cette variante présente l'avantage de chauffer uniformément le mélange gazeux contenu dans chaque canal.

De manière préférée, le dispositif d'analyse est réalisé par des techniques de la microélectronique et il se présente sous la forme d'une puce dont les plots de contact sont reliés à un système de contrôle et de mesure UC (figure 1) comportant les moyens d'alimentation électrique des conducteurs électriques. Sur la figure 1, sont schématisés les plots de contact 10 destinés à connecter les conducteurs électriques au système de contrôle et de mesure UC. Dans l'exemple représenté, ils sont formés, par exemple par dépôt sur le substrat inférieur. Mais à la fois le substrat supérieur et le substrat inférieur ou le substrat inférieur uniquement pourrai(en)t comporter des plots de contact.

De manière avantageuse, des moyens de chauffage sont prévus pour chauffer le canal microfluidique à une température donnée, par exemple par dépôt de conducteur métallique sur une des faces du substrat inférieur ou supérieur de la puce. Les moyens de chauffage sont par exemple de type à effet Joule.

Des moyens de mesure de la température 12 du substrat dans lequel est réalisé le canal sont avantageusement prévus afin de connaître la température des parois du canal lors des mesures et afin de pouvoir en tenir compte lors du traitement des mesures.

La mise en oeuvre d'un canal microfluidique, i.e. présentant une faible section permet l'apparition de différents tronçons de gaz thermalisés à des températures tout en disposant d'un dispositif compact. En effet la faible section du canal permet de localiser les échauffements de gaz seulement autour de chaque membrane.

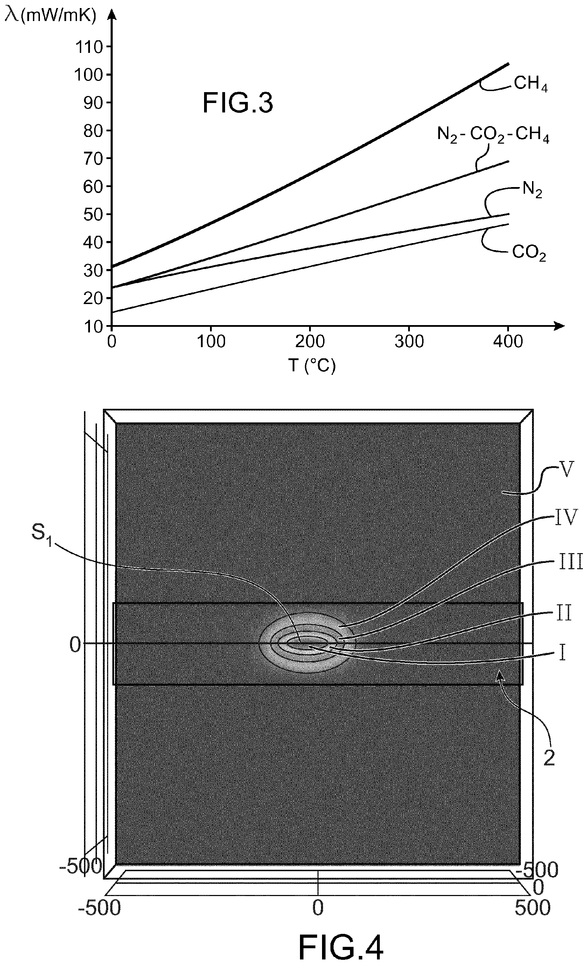

Sur la figure 4, on peut voir représentée schématiquement une simulation de l'échauffement du volume de gaz autour d'une membrane S1 carrée de 150 $\mu$m de côté suspendue dans un canal micro-fluidique de section transversal 200$\mu$mx200$\mu$m et de 1000 $\mu$m de longueur. La membrane est plongée dans un gaz, par exemple de l'air, au sein du canal. Une puissance de 0,125 W est utilisée, par exemple obtenue avec une tension de 5V arrivant sur une résistance de 200 $\Omega$. Les surfaces externes du substrat dans lequel est gravé le canal sont maintenues à température ambiante. La simulation montre que dans le volume de gaz localisé autour de la membrane, typiquement dans une zone s'étendant entre 50 $\mu$m au-dessous et 50 $\mu$m en-dessous, la température peut atteindre des températures supérieures à 500 °C.

La zone I correspond à une température supérieure à 800°C.

La zone II correspond à une température entre environ 600°C et 800°C.

La zone III correspond à une température entre environ 400°C et 600°C.

La zone IV correspond à une température entre environ 200°C et 400°C.

La zone V correspond à une température inférieure à 200°C.

Cette simulation montre donc que, dans le dispositif selon l'invention, la température du volume de gaz autour de l'élément suspendu peut localement atteindre plusieurs centaines de degrés en utilisant une puissance de l'ordre de 0,1 W. De plus, elle montre que l'élévation de température est localisée autour de la membrane, typiquement sur une distance de 100 $\mu$m, et qu'en disposant les membranes à plusieurs centaines de micron les unes des autres, il peut être considéré que chaque membrane a son environnement thermique propre.

Il est alors possible de chauffer à des températures différentes chaque volume de gaz autour de chaque élément suspendu et ainsi de déterminer simultanément la conductivité du mélange gazeux à différentes températures ou un autre paramètre lié aux échanges thermiques et dépendant de la composition du mélange gazeux.

De préférence, dans le cas d'un canal de section transversale carrée la distance entre deux bords en regard de deux membranes est de l'ordre d'une longueur d'un côté du canal. Par exemple, dans le cas d'un canal de 200$\mu$mx200$\mu$m, la distance entre deux bords en regard de deux membranes successives est de 200 $\mu$m.

La distance entre deux bords en regard de deux membranes successives est choisie notamment en fonction de la section du canal. En effet on constate, que la distance sur laquelle le gaz est échauffé autour de la membrane est environ d'une demi-hauteur de canal tout autour du canal. Typiquement, la distance entre les deux bords en regard de deux membranes successives est égale à environ une hauteur du canal. Par exemple dans le cas d'un canal microfluidique de 200$\mu$m $\times$ 200 $\mu$m, la distance entre les deux bords en regard de deux membranes successives est de l'ordre de 200 $\mu$m.

Le canal microfluidique a par exemple une hauteur et une largeur comprises entre 50 $\mu$m et 1000 $\mu$m et une longueur comprise entre 500 $\mu$m et 10000 $\mu$m. De préférence, le canal présente une longueur et une largeur inférieure à 500 $\mu$m, typiquement égales à 200 $\mu$m. Cette faible section permet de porter quasiment instantanément un volume de gaz autour de l'élément suspendu à une température de consigne Ti et de minimiser la consommation du dispositif. Elle permet également de plus de localiser l'échauffement de gaz autour de la membrane sur une distance de l'ordre de 100 $\mu$m.

Les membranes ont par exemple une longueur et une largeur comprises entre 2000 $\mu$m² et 200000 $\mu$m²

Par exemple pour une membrane rectangulaire, elle peut avoir la longueur d'un côté comprise entre 10 $\mu$m et 200 $\mu$m et la longueur de l'autre bord comprise ente 200 $\mu$m et 1000 $\mu$m.

Les bras de suspension ont par exemple une longueur de plusieurs dizaines de $\mu$m, par exemple entre 10 $\mu$m et 100 $\mu$m une largeur de quelque $\mu$m à quelques dizaines de $\mu$m, par exemple entre 15 $\mu$m et 30 $\mu$m.

Les membranes sont avantageusement en SiN qui présente une conductivité thermique 60 fois plus faible que le silicium. Les conducteurs électriques peuvent être tout matériau conducteur, par exemple en platine.

Dans le premier mode de réalisation, les éléments suspendus sont de préférence des membranes suspendues. En variante néanmoins, les membranes pourraient être remplacées par des fils conducteurs.

En variante encore, chaque volume de gaz pourrait comporter un élément de chauffage avantageusement formé par un fil conducteur et un élément de mesure, avantageusement formé par un fil conducteur.

Le fonctionnement du dispositif d'analyse va maintenant être décrit.

Le mélange gazeux comportant au plus n gaz connus est introduit dans le canal à l'entrée d'alimentation 2.1.

Dans un mode de fonctionnement, la sortie d'évacuation 2.2 est obturée et le mélange gazeux est statique dans le canal lors des mesures.

Dans un autre mode de fonctionnement, le gaz s'écoule dans le canal entre l'entrée 2.1 et la sortie 2.2 pendant les mesures mais l'écoulement est tel qu'il ne crée pas de turbulences au niveau des éléments suspendus.

Dans un autre mode de fonctionnement, les entrées du capot ou canal sont ouvertes et le gaz entre dans la puce par diffusion ou convection.

Selon un exemple de fonctionnement avantageux, le système de contrôle et de mesure alimente individuellement les conducteurs électriques avec une tension continue de sorte à chauffer le volume de mélange gazeux Vi à une température de travail Ti. Vu le faible volume de gaz considéré, cette températures de travail est atteinte très rapidement, typiquement en un temps inférieur à 1 ms pour chaque volume Vi. Le système de contrôle et de mesure alimentent également le conducteurs électriques avec un signal alternatif de sorte à imposer une excursion de température à l'élément suspendu Si autour de la température de travail Ti. Les membranes étant entourées du mélange gazeux à Ti, elles échangent de la chaleur avec le mélange gazeux.

Le système de contrôle et de mesure mesure l'intensité aux bornes du conducteur électrique pour connaître sa thermorésistance et en déduire la température de l'élément suspendu. La connaissance de cette température permet de déterminer la conductivité thermique $\lambda i(Ti)$.

La température peut croître d'un élément suspendu à l'autre de manière monotone ou les températures des éléments suspendues peuvent être réparties aléatoirement.

Comme montré ci-dessus, les éléments suspendus étant suffisamment petits et étant suffisamment éloignés les uns des autres, le chauffage délivré par le conducteur électrique de l'un des éléments suspendus n'influe pas ou alors très peu sur la température des zones de gaz voisines et des éléments suspendus voisins. Il est alors possible de réaliser simultanément les mesures sur tous les éléments suspendus et à des températures différentes.

On peut ensuite utiliser l'équation de Wassiljewa ci-dessous qui permet d'approximer la conductivité thermique d'un mélange de gaz

$$\lambda m = \sum_{i=1}^{n} \frac{xi\lambda i}{\sum_{j=1}^{n} xiAij}$$

Avec $\lambda m$ la conductivité thermique du mélange, $\lambda i$ la conductivité thermique du composant i,
xi la concentration du composé i, et Aij une fonction qui est par exemple donnée dans le document A micromachined thermoelectric sensor for natural gas analysis: Thermal model and experimental results" Udina, S., Carmona, M., Caries, G., Santander, J., Fonseca, L., Marco, S. (2008) Sensors and Actuators, B: Chemical, 134 (2), pp. 551-558

Chaque élément suspendu fonctionnant autour de T1, ..., Tn-1 permet alors de déterminer la conductivité thermique du mélange gazeux à chaque température T1, ..., Tn-1, i.e. $\lambda m(T1),... \lambda m(Tn)$. Connaissant en outre les valeurs de conductivité thermique de chacun des n gaz du mélange gazeux aux températures T1, ..., Tn-1, on peut résoudre le système de n équations à n inconnus:

$$\begin{cases} x_1 + \cdots + x_n = 1 \\ \lambda(x_1, \ldots, x_n, T_1, P_0) = m_1 \\ \ldots \\ \lambda(x_1, \ldots, x_n, T_{n-1}, P_0) = m_{n-1} \end{cases}$$

On obtient alors les fractions $x_1...x_n$.

De façon alternative à l'utilisation de l'équation de Wassiljewa, il est possible de remonter à la composition du mélange gazeux à partir des mesures effectuées après avoir procédé à une première étape de calibration. Cette étape de calibration consiste, sur un banc permettant de mélanger les gaz de façon calibrée, à mesurer les réponses des n-1 éléments suspendus (de type tension, courant ou conductivité thermique) sur plusieurs points du domaine de composition du mélange d'intérêt. Par des méthodes de traitements des données (par exemple par interpolation), il est possible alors d'estimer les réponses des n-1 éléments entre les points utilisés pour la calibration.

Le fonctionnement d'un dispositif d'analyse dans lequel les éléments suspendus sont des fils suspendus est similaire à celui décrit ci-dessus. Le fil chauffant sert à la fois à chauffer le volume de gaz l'entourant et aux mesures. Dans le cas où chaque volume à un élément chauffant et un élément de mesure, le signal fourni par l'élément de mesure renseigne sur la conductivité thermique du mélange gazeux entre l'élément de chauffage et l'élément de mesure.

Sur la figure 3, on peut voir une représentation graphique de la variation de la conductivité thermique $\lambda$ en mW/mK du $N_2$, du $CO_2$, du méthane et d'un mélange de $N_2$, $CO_2$ et méthane en fonction de la température T en °C. Ces variations des conductivités sont connues.

On constate qu'une mesure à 20°C ne permet pas de distinguer le $N_2$ et le mélange.

Grâce au dispositif selon l'invention, à partir des mesures de conductivités thermiques du mélange gazeux et de la connaissance des conductivités thermiques des composés du mélange aux températures imposés dans chaque section du canal, il est possible de distinguer le $N_2$ du mélange et ceci de manière rapide.

Selon une variante avantageuse, on peut envisager que chaque élément suspendu soit monté en pont de Wheatstone avec élément disposé à l'extérieur du canal. Un tel montage permet de supprimer l'influence de la variation de la température extérieure sur les mesures.

En variante, on pourrait envisager de mettre en oeuvre deux éléments suspendu dans le même tronçon de gaz, les deux éléments étant montés en pont de Wheatstone.

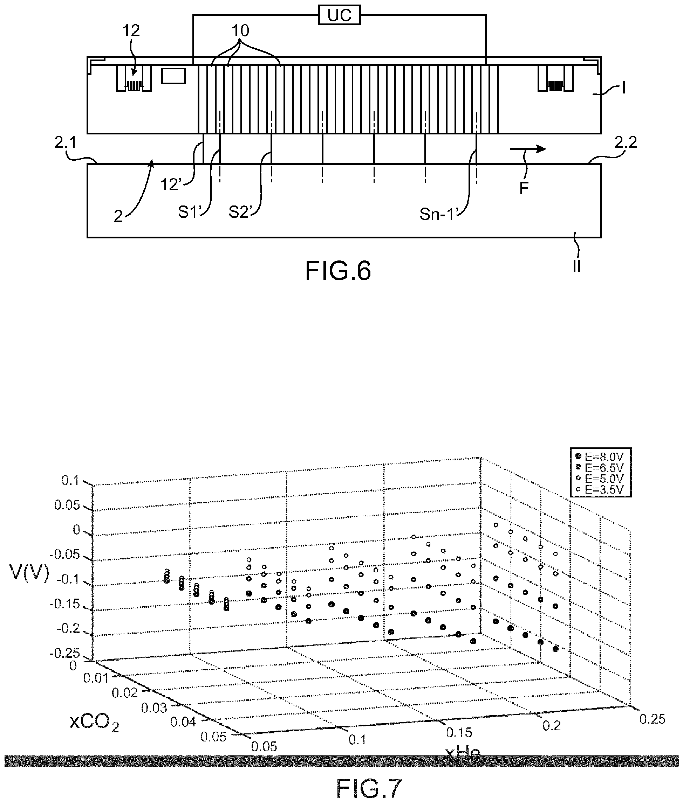

Selon un autre mode de réalisation, on met en ouvre des moyens de chauffage commun à tous les éléments suspendus comme cela est schématisé sur la figure 6.

Dans un exemple de réalisation, les moyens de chauffage 12' comportent un fil conducteur produisant de la chaleur par effet Joule disposé à une extrémité du canal, les éléments suspendus S1', S2'....Sn.l', avantageusement des fils conducteurs sont répartis dans le canal. Chaque élément suspendu se trouve alors chauffé à une température donnée en fonction de la puissance thermique dissipée par le fil chauffant et de son emplacement par rapport au fil chauffant. Cette température donnée est par exemple déterminée lors de la calibration du dispositif. On définit ainsi des tronçons de gaz à des températures données.

Dans un autre exemple, les moyens de chauffage imposent un gradient donné aux éléments suspendus. Par exemple les moyens de chauffage comportent un fil chauffant à une extrémité du canal et un fil chauffant à l'autre extrémité du canal, les éléments suspendus étant suspendus entre les deux fils chauffant. En alimentant en courant les deux fils chauffants, on impose un gradient thermique entre les deux fils chauffant. Chaque élément suspendu est alors à une température donnée en fonction de son emplacement entre les deux fils chauffants. On définit ainsi des tronçons de gaz à des températures données.

Dans ce deuxième mode de réalisation, les éléments suspendus sont de préférence des fils conducteurs.

Les fils sont suspendus transversalement dans le canal. Les fils sont de préférence parallèles à un plan de section longitudinale du canal.

Les fils suspendus ont par exemple une largeur et une épaisseur comprise entre 1 et 20 $\mu$m et une longueur comprise entre 100-1000 $\mu$m, i.e. la largeur du canal.

Le fonctionnement de ce mode de réalisation va maintenant être décrit dans le cas des moyens de chauffage à deux fils chauffants.

Les deux fils chauffants sont alimentés électriquement par exemple en continu de sorte à créer un gradient thermique entre eux. Chaque tronçon de gaz entourant chaque élément suspendu est à une température donnée. En appliquant une tension aux bornes de chaque élément suspendu, on peut mesurer l'intensité qui dépend de sa résistance électrique et donc de la température. Or la température dépend de la composition du mélange gazeux à la température donnée entourant l'élément suspendu. En mesurant l'intensité aux bornes des éléments suspendus dans les différents tronçons de gaz à différentes températures, on peut remonter à la composition du mélange gazeux.

En variante, on pourrait alimenter les moyens de chauffage en tension alternative.

On peut faire une analogie entre cet exemple de mesure et la technique de mesure quatre points d'une résistance électrique avec: la température qui serait analogue à la tension électrique, le flux de chaleur qui serait analogue au courant électrique, la résistance thermique qui serait analogue à la résistance électrique, la capacité thermique qui serait analogue à la capacité électrique, le conducteur thermique formé par le microcanal de gaz et le substrat qui seraient analogues au conducteur électrique, la sonde de température serait analogue au voltmètre et la source thermique serait analogue à la source de courant.

Dans ce mode de réalisation, les fils de mesure sont sous l'influence thermique du ou des fils chauffants. De préférence, les fils de mesure se trouvent à une distance du ou des fils chauffants inférieure ou égale à la longueur d'un côté du canal, par exemple ils se trouvent au plus à 200 $\mu$m du fil chauffant pour un canal de 200 $\mu$m $\times$ 200 $\mu$m.

Dans les exemples ci-dessus, le paramètre du mélange de gaz et de chacun des gaz considéré pour les analyser est la conductivité thermique. Mais d'autres paramètres physiques aptes à caractériser un gaz ou un mélange de gaz peuvent être utilisés tels que la densité, la viscosité, la vitesse du son...

A titre d'exemple uniquement, nous donnons un exemple de dimensionnements d'un dispositif d'analyse selon l'invention

Les détecteurs de conductivité thermique ou TCD (Thermal Conductivity Detector en terminologie anglo-saxonne) ont typiquement une limite de détection et précision de l'ordre de 1 ppm, en utilisant le contraste de conductivité thermique avec un gaz vecteur à « haute conductivité thermique » comme l'Helium par exemple (152,0 mW·m$^{-1}$·K$^{-1}$). Dans le cas du dispositif d'analyse selon l'invention, la limite de détection et précision peut atteindre 1 ppm. Les rapports des conductivités thermiques composant les gaz à analyser pourront cependant être 10 à 100 fois moindres que ceux obtenus avec l'Helium, dégradant la limite de détection d'autant. En effet, pour un mélange de deux gaz A et B de conductivité thermique différente, la variation du signal sur la pleine échelle de mesure est en première approximation d'autant plus grande que l'écart des conductivités thermiques entre A et B est grande. A niveau de bruit égal sur la mesure, les limites de détection en concentration seront donc moins bonnes lorsque l'écart (et donc la pleine échelle de mesure) entre les conductivités thermiques diminue.

Le dispositif d'analyse peut comporter des conducteurs électriques ayant une résistance de l'ordre de 100 Ohms. Sous une tension de 5V, ils permettent de thermaliser localement le gaz à des températures supérieures à 500 °C. La puissance dissipée dans une telle membrane reste cependant faible 5$\times$5/100 W = 0,25 W. La puissance nécessaire pour le capteur et le microcontroleur sera donc de quelques Watts, puissance qui est par exemple compatible avec une alimentation par port USB qui délivre une puissance entre 5W et 10W.

Le volume interne peut être de 100$\mu$m$\times$100$\mu$m$\times$10000$\mu$m, soit 0,1 $\mu$L.

Un débit de 1 mL/min permet de remplir la cavité d'analyse en un temps inférieur à 1 ms. La faible capacité thermique des membranes et du gaz environnant permet un thermalisation rapide en des temps de l'ordre ou inférieur à 1ms. Ces temps de réponse du capteur permettent des analyses en un temps inférieur à 10s.

Un tel dispositif a un coût estimé inférieur à 100€, une masse inférieure à 500g et un volume externe de l'ordre de 5 cm$^2$.

Le dispositif d'analyse selon l'invention peut donc présenter un encombrement réduit, un coût de revient limité, un temps d'analyse très faible et une consommation électrique réduite.

De manière très avantageuse, le dispositif d'analyse peut remplacer avantageusement des systèmes d'analyse à colonne de chromatographie en phase gazeuse ou à des capteurs de mélange de gaz binaire ou être associé à une colonne de chromatographie.

Nous allons maintenant décrire une calibration d'un dispositif selon l'invention et de mesure de la composition d'un axe.

On considère le mélange de gaz $CO_2$, He, $N_2$.

Le dispositif utilisé comporte deux membranes suspendues par tronçon de gaz à température donnée, les mesures sur les deux membranes étant moyennées.

On note E la tension d'entrée appliquée à la membrane. On note V, la tension de sortie liée à la mesure de la résistance ou du courant traversant le fil thermorésistif de la membrane. Il s'agit par exemple d'un montage de type : mesure de tension au borne d'une résistance en série avec la membrane étudiée ; ou montage de type pont de Wheatstone. Afin de sonder le gaz à différentes températures, différentes tensions d'entrée sont appliquées sur les membranes. Dans l'exemple représenté, les valeurs de tension d'entrée sont appliquées : 3.5V, 5V, 6.5V, 8V.

Le dispositif est d'abord calibré sur un domaine défini de composition. Pour cela, le dispositif est placé à la sortie d'un banc gaz qui permet de préparer les différentes compositions du mélange $CO_2$, He, $N_2$. Pour chacune de ces compositions, les tensions de sorties des différentes membranes sont mesurées. L'ensemble des résultats expérimentaux de calibration pour le $CO_2$ et He est représenté dans la figure 7. La fraction de N2 est obtenue en faisant $1-x_{CO2}-x_{He}$.

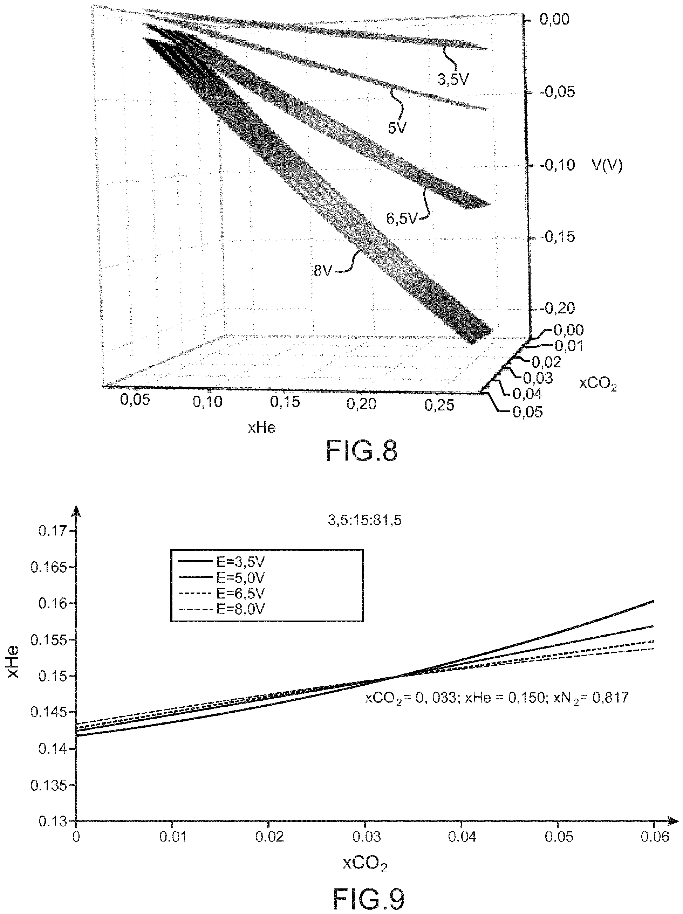

Ces résultats expérimentaux sont ensuite interpolés à l'ordre 2 sur le domaine de calibration. Cette interpolation aboutit à la définition des quatre surfaces représentées dans la figure 8 et définies par les quatre équations pour chacune des tensions d'entrée.

$$(x_{CO2}, x_{He}) \rightarrow V_{E=3.5V}(x_{CO2}, x_{He})$$
$$(x_{CO2}, x_{He}) \rightarrow V_{E=5V}(x_{CO2}, x_{He})$$
$$(x_{CO2}, x_{He}) \rightarrow V_{E=6.5V}(x_{CO2}, x_{He})$$
$$(x_{CO2}, x_{He}) \rightarrow V_{E=8V}(x_{CO2}, x_{He})$$

Le dispositif ayant été calibré et disposant des représentations graphiques reliant la tension de sortie aux fractions de gaz en fonction de différentes tensions d'entrée, nous analysons un mélange gazeux comportant $CO_2$, He, $N_2$ de composition connue afin de vérifier les performances du dispositif. La concentration de chacun des gaz est notée $x_{CO2}$, $x_{He}$, $x_{N2}$. La composition du mélange gazeux est 3.5% de CO2, 15% d'Hélium, et 81.5% d'azote.

Le mélange de gaz est introduit dans le canal microfluidique du dispositif. Celui-ci nous donne des mesures expérimentales des tensions de sorties pour chacune des tensions d'entrées. A partir de la représentation graphique de la figure 8, il est possible de tracer quatre droites représentées sur la figure 9.

Nous montrons ici graphiquement comment procéder pour remonter à une estimation de la composition du gaz ternaire à partir de ces données du capteur et des surfaces de calibration.

Pour déterminer $x_{CO2}$ et $x_{He}$, seules deux équations sont nécessaires : le couple $x_{CO2}$ et $x_{He}$ étant alors défini à l'intersection des deux courbes correspondantes. $x_{CO2}$ et $x_{He}$ étant alors déterminés, on peut déterminer $x_{N2}$ car $x_{CO2} + x_{He} + x_{N2} = 1$.

Nous constatons ici, aves les deux mesures supplémentaires, que les courbes se recoupent en un point proche de $x_{CO2}$ = 3.3%, $x_{He}$=15%, $x_{N2}$=81.7%.

On constate une erreur de mesure de composition inférieure à 1% par rapport à la composition du mélange. Le dispositif selon l'invention permet donc de réaliser en une seule opération la composition d'un mélange gazeux comportant trois composés de manière performante.

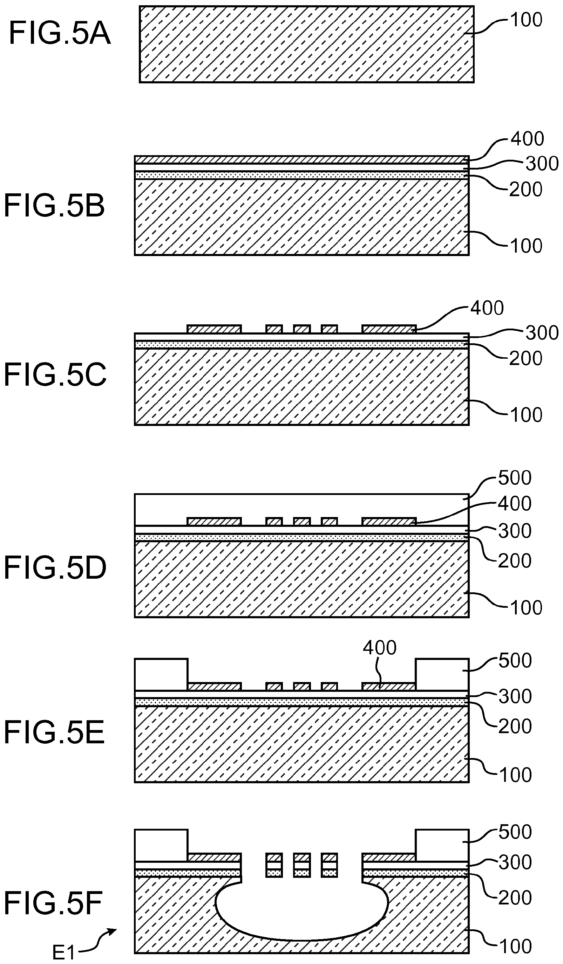

Un exemple de procédé de réalisation va être décrit en relation avec les figures 5A à 5K.

On part par exemple d'un substrat 100 en matériau semi-conducteur par exemple en Si ayant par exemple une épaisseur de 540 μm.

Celui-ci est représenté sur la figure 5A.

On forme ensuite une couche d'oxyde 200, par exemple $SiO_2$ par exemple de 50 nm d'épaisseur, par exemple par oxydation thermique, puis une couche de SiN 300 par exemple de 200 nm d'épaisseur par exemple par dépôt chimique en phase vapeur, et une couche d'un matériau conducteur électrique 400 par en exemple le Pt, par exemple par pulvérisation, d'épaisseur 100 nm.

L'élément ainsi obtenu est représenté sur la figure 5B.

Lors d'une étape suivante, on structure la couche 400, par exemple par photolithographie et gravure ionique. La résine résiduelle de l'étape de photolithographie est ensuite retirée.

L'élément ainsi obtenu est représenté sur la figure 5C.

Lors d'une étape suivante, on forme une couche d'oxyde 500 sur la couche 400 structurée, et ayant par exemple une épaisseur de 500 nm, par exemple par dépôt chimique en phase vapeur.

L'élément ainsi obtenu est représenté sur la figure 5D.

Lors d'une étape suivante, on structure la couche 500 par exemple par photolithographie, gravure avec arrêt sur la couche 300 de SiN. La résine résiduelle de l'étape de photolithographie est ensuite retirée.

L'élément ainsi obtenu est représenté sur la figure 5E.

Lors d'une étape suivante on structure le substrat 100 par exemple par photolithographie, gravure des couches 300 et 200 par exemple par gravure sèche anisotrope et isotrope. La résine résiduelle de l'étape de photolithographie est ensuite retirée.

L'élément ainsi obtenu, désigné E1, est représenté sur la figure 5F. Il comporte des éléments suspendus.

Lors d'une étape suivante on réalise un capot E2. On utilise par exemple un substrat en verre 600 sur lequel on forme une couche de W/WN/Au, qui servira de masque, par exemple par dépôt par exemple par pulvérisation, ayant par exemple une épaisseur de 200 nm.

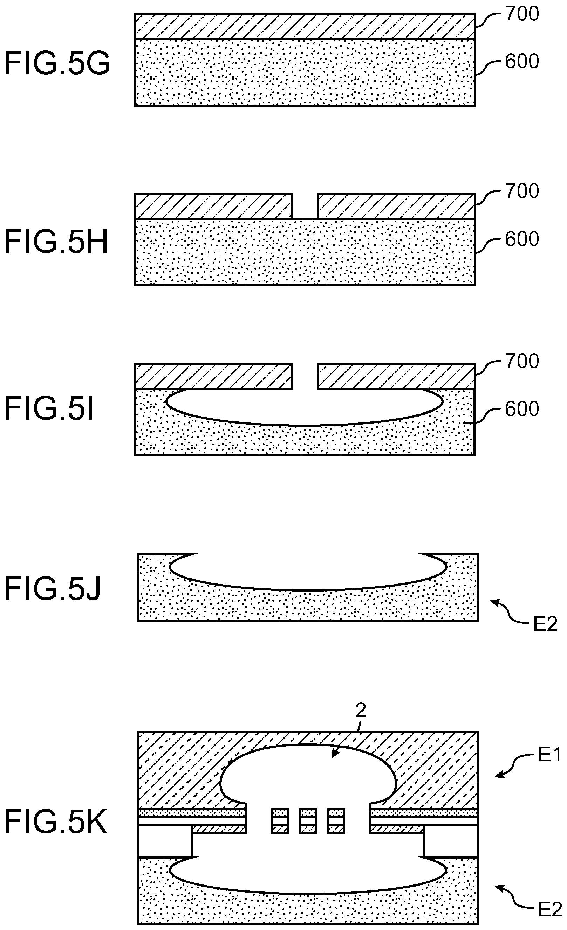

L'élément ainsi obtenu est représenté sur la figure 5G.

Lors d'une étape suivante, on structure la couche 600, par exemple par photolithographie et gravure de la couche 700 par exemple par gravure humide HF. La résine résiduelle de l'étape de photolithographie est ensuite retirée.

L'élément ainsi obtenu est représenté sur la figure 5H.

Lors d'une étape suivante, on réalise une gravure du substrat 600 au HF.

L'élément ainsi obtenu est représenté sur la figure 5I.

Lors d'une épate suivante, le masque 700 est retiré.

L'élément ainsi obtenu est représenté sur la figure 5J. On obtient l'élément E2 qui va former un capot.

Lors d'une étape suivante, on assemble les éléments E1 et E2, par exemple par scellement anodique. On délimite ainsi un canal microfluidique dans lequel sont suspendus des éléments S1, S2, S3.

L'élément ainsi obtenu est représenté sur la figure 5K.

## Revendications

1. Dispositif d'analyse pour déterminer des concentrations de n gaz d'un mélange gazeux, comportant un canal microfluidique (2) s'étendant entre une première extrémité et une deuxième extrémité, au moins n-1 éléments suspendus dans le canal microfluidique (2), le nombre d'élément suspendus étant au moins égal à 2, lesdits n-1 éléments (S1, S2... Sn-1) se succédant de la première extrémité du canal microfluidique (2) vers la deuxième extrémité du canal microfluidique (2), des moyens de chauffage aptes à chauffer une zone du mélange gazeux entourant chaque élément suspendu (S1, S2,...Sn-1), les moyens de chauffage et la disposition des éléments suspendus étant tels que chaque zone du mélange gazeux est chauffée à une température donnée différente de celles auxquelles sont chauffées les zones de mélange gazeux entourant les autres éléments suspendus (S1, S2... Sn-1), des moyens de mesure de la température ou de la variation de température de chaque élément suspendu (S1, S2... Sn-1) et un système de contrôle et de mesure (UC) connectés aux moyens de chauffage et aux moyens de mesure, dans lequel le système de contrôle et de mesure commande les moyens de chauffage de sorte que chaque zone du mélange gazeux est chauffée à une température donnée différente de celles auxquelles sont chauffées les autres zones du mélange gazeux , et dans lequel le système de contrôle et de mesure est configuré pour utiliser la température ou la variation de température de n-1 éléments (S1, S2... Sn-1) pour déterminer n-1 conductivités thermiques associées aux n-1 températures ou variations de températures mesurées, et déterminer les concentrations de n gaz.

2. Dispositif d'analyse selon la revendication 1, dans lequel chaque volume de mélange gazeux comporte ses moyens de chauffage individuels.

3. Dispositif d'analyse selon la revendication 2, dans lequel le conduit a une section transversale rectangulaire présentant une hauteur et une largeur, la distance entre deux bords en regard de deux éléments suspendus successifs est de l'ordre de la hauteur du canal.

4. Dispositif d'analyse selon la revendication 2 ou 3, dans lequel la distance entre deux bords en regard de deux éléments suspendus successifs est comprise entre 200$\mu$m et 2000$\mu$m.

5. Dispositif d'analyse selon l'une des revendications 1 à 4, dans lequel chaque élément suspendu comporte une membrane suspendue par des bras de suspension dans le canal et dans lequel les moyens de chauffage comportent un fil conducteur formé sur une face de la membrane, la membrane ayant avantageusement des surfaces comprises entre 200 $\mu$m$^2$ et 200000 $\mu$m$^2$ et/ou les bras de suspension ayant avantageusement une longueur de plusieurs dizaines de $\mu$m et une largeur de quelques $\mu$m à quelques dizaines de $\mu$m.

6. Dispositif d'analyse selon l'une des revendications 1 à 4, dans lequel chaque élément suspendu comporte au moins un fil suspendu, le fil suspendu formant avantageusement les moyens de chauffage et les moyens de mesure de température ou de la variation de température.

7. Dispositif d'analyse selon la revendication 2, comportant n-1 puces comportant un sous-canal fluidique disposées dans le canal fluidique du dispositif, le volume intérieure des sous-canaux fluidiques étant destinés à contenir une zone de mélange gazeux, chaque élément suspendu étant suspendu dans un sous-canal fluidique, les moyens de chauffage étant situés sur une face extérieure de chaque puce.

8. Dispositif d'analyse selon la revendication 1, dans lequel les moyens de chauffage sont communs à tous les volumes de mélange gazeux, les moyens de chauffage comportant avantageusement au moins un fil conducteur destiné à chauffer tous les volumes de mélange gazeux ou les moyens de chauffage étant disposés de part et d'autres des éléments suspendus de sorte à imposer un gradient thermique au mélange gazeux situé entre les moyens de chauffage.

9. Dispositif d'analyse selon la revendication 8, dans lequel le conduit a une section transversale rectangulaire présentant une hauteur et une largeur et dans lequel la distance entre les moyens de chauffage et les moyens de mesure est inférieure ou à la hauteur du canal.

10. Dispositif d'analyse selon la revendication 8 ou 9, dans lequel les moyens de chauffage et les moyens de mesure comportent des fils conducteurs électriques suspendus dans le canal, le fil suspendu ayant avantageusement une section transversale comprise entre 1 $\mu$m et 20 $\mu$m et une longueur comprise entre 100 $\mu$m et 1000 $\mu$m.

11. Dispositif d'analyse selon l'une des revendications 1 à 10, dans lequel la section du canal fluidique est comprise entre 100 $\mu$m$^2$ et 1 mm$^2$.

12. Dispositif d'analyse selon l'une des revendications 1 à 11, dans lequel chaque élément suspendu est monté en pont de Wheatstone avec au moins un autre élément suspendu disposé à l'extérieur du canal microfluidique.

13. Dispositif d'analyse selon l'une des revendications 1 à 12, dans lequel le système de contrôle et de mesure de la température comporte des moyens d'alimentation électrique aptes à appliquer un signal continu permettant le chauffage du mélange gazeux et un signal alternatif permettant la mesure de la température ou variation de température.

14. Procédé d'analyse d'un mélange gazeux de n gaz, comprenant les étapes :

a) Introduction du mélange gazeux comportant n composants connus dans un canal fluidique, le canal fluidique comportant au moins n-1 éléments suspendus dans le canal, le nombre d'élément suspendus étant au moins égal à 2, lesdits éléments suspendus étant disposés les uns à côté des autres dans une direction d'écoulement du mélange gazeux dans le canal microfluidique,
b) Chauffage d'un volume de gaz entourant chaque élément suspendu à une température donnée différente de celles des autres volumes de gaz,
c) Mesure de la température ou la variation de température de n-1 éléments (S1, S2... Sn-1) pour déterminer n-1 conductivités thermiques associées aux n-1 températures ou variations de températures mesurées,d) Détermination d'une concentration xj de chaque composant du mélange de gaz à partir des n-1 mesures obtenues à l'étape c).

15. Procédé d'analyse d'un mélange gazeux de n gaz selon la revendication 14, dans lequel l'étape b) a lieu en chauffant l'élément suspendu à une température proche de celle à laquelle est chauffé le volume de gaz entourant ledit élément suspendu, avantageusement l'étape a) ayant lieu en appliquant une tension continu à un conducteur électrique et dans lequel l'étape b) ayant lieu en appliquant une tension alternative au conducteur électrique ou à un autre conducteur électrique.

**Patentansprüche**

1. Analysevorrichtung zur Bestimmung der Konzentrationen von n Gasen einer Gasmischung, umfassend einen Mikrofluidkanal (2), der sich zwischen einem ersten Ende und einem zweiten Ende erstreckt, wenigstens n-1 Elemente, die in dem Mikrofluidkanal (2) aufgehängt sind, wobei die Zahl von aufgehängten Elementen wenigstens gleich 2 ist, wobei die n-1 Elemente (S1, S2... Sn-1) von dem ersten Ende des Mikrofluidkanals (2) in Richtung des zweiten Endes des Mikrofluidkanals (2) aufeinander folgen, Heizmittel, die dazu ausgelegt sind, eine Zone der Gasmischung zu heizen, die jedes aufgehängte Element (S1, S2, ...Sn-1) umgibt, wobei die Heizmittel und die Anordnung der aufgehängten Elemente derart sind, dass jede Zone der Gasmischung auf eine gegebene Temperatur geheizt wird, die verschieden ist von jenen, auf die die Zonen der Gasmischung geheizt werden, die die anderen aufgehängten Elemente (S1, S2... Sn-1) umgeben, Mittel zum Messen der Temperatur oder der Temperaturvariation jedes aufgehängten Elements (S1, S2... Sn-1) und ein System zur Steuerung und zur Messung (UC), die mit den Heizmitteln und mit den Messmitteln verbunden sind, wobei das Steuer- und Messsystem die Heizmittel derart steuert, dass jede Zone der Gasmischung auf eine gegebene Temperatur geheizt wird, die von jenen verschieden ist, auf die die anderen Zonen der Gasmischung geheizt werden, und wobei das Steuer- und Messsystem dazu konfiguriert ist, die Temperatur oder die Temperaturvariation von n-1 Elementen (S1, S2... Sn-1) zu verwenden, um n-1 thermische Leitfähigkeiten zu bestimmen, die den n-1 gemessenen Temperaturen oder Temperaturvariationen zugeordnet sind, und die Konzentrationen von n Gasen zu bestimmen.

2. Analysevorrichtung nach Anspruch 1, bei der jedes Volumen der Gasmischung seine individuellen Heizmittel umfasst.

3. Analysevorrichtung nach Anspruch 2, bei der die Leitung einen rechteckigen Querschnitt hat, der eine Höhe und eine Breite aufweist, wobei der Abstand zwischen zwei gegenüberliegenden Rändern von zwei aufeinanderfolgenden aufgehängten Elementen in der Größenordnung der Höhe des Kanals ist.

4. Analysevorrichtung nach Anspruch 2 oder 3, bei der der Abstand zwischen zwei gegenüber liegenden Rändern von zwei aufeinanderfolgenden aufgehängten Elementen zwischen 200 $\mu$m und 2000 $\mu$m enthalten ist.

5. Analysevorrichtung nach einem der Ansprüche 1 bis 4, bei der jedes aufgehängte Element eine Membran umfasst, die mittels Aufhängungsarmen in dem Kanal aufgehängt ist, und bei der die Heizmittel einen leitenden Draht umfassen, der auf einer Fläche der Membran gebildet ist, wobei die Membran vorzugsweise Oberflächen hat, die zwischen 200 $\mu$m$^2$ und 200000 $\mu$m$^2$ enthalten sind, und/oder wobei die Aufhängungsarme vorzugsweise eine Länge von mehreren Zehn $\mu$m und eine Breite von einigen $\mu$m bis einigen Zehn $\mu$m haben.

6. Analysevorrichtung nach einem der Ansprüche 1 bis 4, bei der jedes aufgehängte Element wenigstens einen aufgehängten Draht umfasst, wobei der aufgehängte Draht vorzugsweise die Heizmittel und die Mittel zum Messen der Temperatur oder der Temperaturvariation bildet.

7. Analysevorrichtung nach Anspruch 2, umfassend n-1 Chips, die einen Fluidunterkanal umfassen, die in dem Fluidkanal der Vorrichtung angeordnet sind, wobei das Innenvolumen der Fluidunterkanäle dazu ausgelegt ist, eine Zone der Gasmischung zu enthalten, wobei jedes aufgehängte Element in einem Fluidunterkanal aufgehängt ist, wobei die Heizmittel auf einer Außenfläche jedes Chips angeordnet sind.

8. Analysevorrichtung nach Anspruch 1, bei der die Heizmittel allen Volumina der Gasmischung gemeinsam sind, wobei die Heizmittel vorzugsweise wenigstens einen leitenden Draht umfassen, der dazu ausgelegt ist, alle Volumina der Gasmischung zu heizen, oder wobei die Heizmittel auf beiden Seiten der aufgehängten Elemente derart angeordnet sind, dass sie der Gasmischung, die sich zwischen den Heizmitteln befindet, einen thermischen Gradienten auferlegen.

9. Analysevorrichtung nach Anspruch 8, bei der die Leitung einen rechteckigen Querschnitt hat, der eine Höhe und eine Breite aufweist, und bei der der Abstand zwischen den Heizmitteln und den Messmitteln kleiner als die Höhe des Kanals ist.

10. Analysevorrichtung nach Anspruch 8 oder 9, bei der die Heizmittel und die Messmittel elektrisch leitende Drähte umfassen, die in dem Kanal aufgehängt sind, wobei der aufgehängte Draht vorzugsweise einen Querschnitt hat, der zwischen 1 $\mu$m und 20 $\mu$m enthalten ist, sowie eine Länge, die zwischen 100 $\mu$m und 1000 $\mu$m enthalten ist.

11. Analysevorrichtung nach einem der Ansprüche 1 bis 10, bei der der Querschnitt des Fluidkanals zwischen 100 $\mu$m$^2$ und 1mm$^2$ enthalten ist.

12. Analysevorrichtung nach einem der Ansprüche 1 bis 11, bei der jedes aufgehängte Element in einer Wheatstonebrücke mit wenigstens einem anderen aufgehängten Element montiert ist, das außerhalb des Mikrofluidkanals angeordnet ist.

13. Analysevorrichtung nach einem der Ansprüche 1 bis 12, bei der das System zur Steuerung und zur Messung der Temperatur elektrische Versorgungsmittel umfasst, die dazu ausgelegt sind, ein Gleichstromsignal anzulegen, das das Heizen der Gasmischung erlaubt, sowie ein Wechselstromsignal, das das Messen der Temperatur oder Temperaturvariation erlaubt.

14. Verfahren zur Analyse einer Gasmischung von n Gasen, umfassend die Schritte:

a) Einbringen der Gasmischung, die n bekannte Komponenten hat, in einen Fluidkanal, wobei der Fluidkanal wenigstens n-1 Elemente umfasst, die in dem Kanal aufgehängt sind, wobei die Zahl von aufgehängten Elementen wenigstens gleich 2 ist, wobei die aufgehängten Elemente in einer Strömungsrichtung der Gasmischung in dem Mikrofluidkanal nebeneinander angeordnet sind,

b) Heizen eines Gasvolumens, das jedes aufgehängte Element umgibt, auf eine gegebene Temperatur, die von jenen der anderen Gasvolumina verschieden ist,

c) Messen der Temperatur oder der Temperaturvariation von n-1 Elementen (S1, S2... Sn-1) zur Bestimmung von n-1 thermischen Leitfähigkeiten, die den n-1 gemessenen Temperaturen oder Temperaturvariationen zugeordnet sind,
d) Bestimmen einer Konzentration xj jeder Komponente der Gasmischung ausgehend von den n-1 Messungen, die im Schritt c) erhalten werden.

15. Verfahren zur Analyse einer Gasmischung von n Gasen nach Anspruch 14, bei dem der Schritt b) erfolgt, indem das aufgehängte Element auf eine Temperatur nahe jener aufgeheizt wird, auf die das Gasvolumen aufgeheizt ist, das das aufgehängte Element umgibt, wobei vorzugsweise der Schritt a) erfolgt, indem eine Gleichspannung an einen elektrischen Leiter angelegt wird, und bei dem der Schritt b) erfolgt, indem eine Wechselspannung an den elektrischen Leiter oder an einen anderen elektrischen Leiter angelegt wird.

**Claims**

1. Analysis device for determining the concentrations of n gases of a gaseous mixture, comprising a microfluidic channel (2) extending between a first end and a second end, at least n-1 suspended elements in the microfluidic channel (2), the number of suspended elements being at least equal to 2, said n-1 elements (S1, S2...Sn-1) succeeding each other from the first end of the microfluidic channel (2) to the second end of the microfluidic channel, heating means capable of heating a zone of the gaseous mixture surrounding each suspended element (S1, S2...Sn-1), the heating means and the arrangement of the suspended elements being such that each zone of the gaseous mixture is heated to a given temperature different to those to which are heated the zones of gaseous mixture surrounding the other suspended elements (S1, S2...Sn-1), means of measuring the temperature or the variation in temperature of each suspended element (S1, S2...Sn-1), and a control and measurement system (UC) connected to the heating means and to the measuring means, in which the control system commands the heating means such that each zone of the gaseous mixture is heated to a given temperature different to those to which are heated the other zones of the gaseous mixture, and in which the control and measurement system is configured to use the temperature or the variation in temperature of n-1 suspended elements (S1, S2...Sn-1) to determine the thermal conductivities associated to the n-1 measured temperature or the variation in temperature, and determine the gas concentrations.

2. Analysis device according to claim 1, wherein each volume of gaseous mixture comprises its individual heating means.

3. Analysis device according to claim 2, wherein the conduit has a rectangular transversal section having a height and a width, the distance between two facing edges of two successive suspended elements is of the order of the height of the channel.

4. Analysis device according to claim 2 or 3, wherein the distance between two facing edges of two successive suspended elements is comprised between 200 $\mu$m and 2000 $\mu$m.

5. Analysis device according to one of the claims 1 to 4, wherein each suspended element comprises a membrane suspended by suspension arms in the channel and wherein the heating means comprise a conducting wire formed on one face of the membrane, the membrane having advantageously surface areas comprised between 200 $\mu$m$^2$ and 200000 $\mu$m$^2$ and/or the suspension arms having a length of several tens of $\mu$m and a width of several $\mu$m to several tens of $\mu$m.

6. Analysis device according to one of the claims 1 to 4, wherein each suspended element comprises at least one suspended wire, the suspended wire forming advantageously the heating means and the means of measuring the temperature or the variation in temperature.

7. Analysis device according to claim 2, comprising n-1 chips comprising a fluidic sub-channel arranged in the fluidic channel of the device, the internal volume of the fluidic sub-channels being intended to contain a gaseous mixture zone, each suspended element being suspended in a fluidic sub-channel, the heating means being situated on an external face of each chip.

8. Analysis device according to claim 1, wherein the heating means are common to all the volumes of gaseous mixture, the heating means comprising advantageously at least one conducting wire intended to heat all the volumes of gaseous mixture or the heating means being arranged on either side of the suspended elements so as to impose a thermal gradient on the gaseous mixture situated between the heating means.

9. Analysis device according to claim 8, wherein the conduit has a rectangular transversal section having a height and a width and wherein the distance between the heating means and the measuring means is less than or the height of the channel.

10. Analysis device according to claim 8 or 9, wherein the heating means and the measuring means comprise electrical conducting wires suspended in the channel, the suspended wire having a transversal section comprised between 1 $\mu$m and 20 $\mu$m and a length comprised between 100 $\mu$m and 1000 $\mu$m.

11. Analysis device according to one of the claims 1 to 10, wherein the section of the fluidic channel is comprised between 100 $\mu$m$^2$ and 1 mm$^2$.

12. Analysis device according to one of the claims 1 to 11, wherein each suspended element is mounted in a Wheatstone bridge with at least one other suspended element arranged outside of the microfluidic channel.

13. Analysis device to one of the claims 1 to 12, wherein the system for controlling and measuring the temperature comprises electrical supply means capable of applying a continuous signal enabling the heating of the gaseous mixture and an alternating signal enabling the measurement of the temperature or variation in temperature.

14. Method of analysing a gaseous mixture of n gases, including the steps:

   a) Introducing the gaseous mixture comprising n known components into a fluidic channel, the fluidic channel comprising at least n-1 suspended elements in the channel, the number of suspended elements being at least equal to 2, said suspended elements being arranged next to each other in a direction of flow of the gaseous mixture in the microfluidic channel,
   b) Heating a volume of gas surrounding each suspended element to a given temperature different to those of the other volumes of gas,
   c) Measuring the temperature or the variation in temperature of n-1 suspended elements (S1, S2...Sn-1) to determine the n-1 thermal conductivities associated to the n-1 measured temperature or the variation in temperature,
   d) Determining a concentration xj of each component of the gas mixture from the n-1 measurements obtained at step c).

15. Method of analysing a gaseous mixture of n gases according to claim 14, wherein step b) takes place by heating the suspended element to a temperature close to that to which is heated the volume of gas surrounding said suspended element, advantageously step a) taking place by applying a direct voltage to an electrical conductor and step b) taking place by applying an alternating voltage to the electrical conductor or to another electrical conductor.

FIG.1

FIG.2

FIG.3

FIG.4

FIG.5A
— 100

FIG.5B
— 400
— 300
— 200

— 100

FIG.5C
— 400
— 300
— 200

— 100

FIG.5D
— 500
— 400
— 300
— 200

— 100

FIG.5E
400
— 500
— 300
— 200

— 100

FIG.5F
— 500
— 300
— 200

— 100

E1

FIG.5G

FIG.5H

FIG.5I

FIG.5J

FIG.5K

FIG.6

FIG.7

FIG.8

FIG.9

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4164862 A

**Littérature non-brevet citée dans la description**

- **UDINA, S. ; CARMONA, M. ; CARIES, G. ; SANTANDER, J. ; FONSECA, L. ; MARCO, S.** A micromachined thermoelectric sensor for natural gas analysis: Thermal model and experimental results. *Sensors and Actuators, B: Chemical,* 2008, vol. 134 (2), 551-558